# EUROPEAN PATENT APPLICATION

(11) **EP 4 674 482 A2**
(43) Date of publication of application: **07.01.2026**
(21) Application number: 25215822.5
(22) Date of filing: 24.10.2013
(51) Int. Cl.: A61P 25/28

(54) **PROPHYLACTIC AND/OR THERAPEUTIC AGENT FOR BEHAVIORAL AND PSYCHOLOGICAL SYMPTOMS ASSOCIATED WITH NEURODEGENERATIVE DISEASE OR IMPULSIVE SYMPTOMS ASSOCIATED WITH MENTAL DISEASE CONTAINING BREXPIPRAZOLE OR SALT THEREOF**

(30) Priority: 25.10.2012 US 201261718305 P; 14.03.2013 US 201361782467 P
(62) Divisional of application: 21159914.7
(71) Applicant: Otsuka Pharmaceutical Co., Ltd., Tokyo 101-8535 (JP)
(72) Inventor: Sato, Shinji, Osaka, 540-0021 (JP); Maeda, Kenji, Osaka, 540-0021 (JP); Ishikawa, Dai, Osaka, 540-0021 (JP); Nakamura, Mai, Osaka, 540-0021 (JP)
(74) Representative: Hoffmann Eitle

(57) **Abstract**

The present invention relates to a prophylactic and/or therapeutic agent for behavioral and psychological symptoms associated with neurodegenerative disease or impulsive symptoms associated with mental disease, which contains 7-[4-(4-benzo[b]thiophen-4-yl-piperazin-1-yl)butoxy]-1H-quinolin-2-one or a salt thereof as an active ingredient.

## Description

### Technical Field

The present invention relates to a prophylactic and/or therapeutic agent for behavioral and psychological symptoms associated with neurodegenerative disease or impulsive symptoms associated with mental disease, which contains brexpiprazole or a salt thereof.

### Background Art

Brexpiprazole (OPC-34712), i.e., 7-[4-(4-benzo[b]thiophen-4-yl-piperazin-1-yl)butoxy]-1H-quinolin-2-one, or a salt thereof and a production method thereof are described in patent document 1 (JP-A-2006-316052 (US 2010/0179322 A1)), and are described to have a dopamine D₂ receptor partial agonist activity (D₂ receptor partial agonist activity), a serotonin 5-HT_{2A} receptor antagonist activity (5-HT_{2A} receptor antagonist activity) and an adrenergic α₁ receptor antagonist activity (α₁ receptor antagonist activity) and, in addition thereto, concurrently has a serotonin uptake inhibitory action (or serotonin reuptake inhibitory action), and have a wide treatment spectrum for the central neurological diseases. While this patent document describes that brexpiprazole or a salt thereof is useful for cognitive impairment associated with neurodegenerative diseases such as Alzheimer's disease and the like, it is completely silent on the usefulness for behavioral and psychological symptoms of neurodegenerative diseases or impulsive symptoms associated with mental diseases.

Moreover, it does not describe that brexpiprazole or a salt thereof significantly increased activation of medial prefrontal cortex (ACA: Anterior cingulate area, PL: Prelimbic area, IL: Infralimbic area).

Decreased activity of the medial prefrontal cortex has been reported to be related to behavioral and psychological symptoms of neurodegenerative diseases, and impulsive symptoms of the mental diseases (Bipolar Disord 2009; 11:628-36, J Abnorm Psychol 2013; 122:558-65, Mov Disord 2011; 26:225-33, Pharmacol Biochem Behav 2009; 93:237-47).

Impulsive symptoms are among the multidimensional personality characteristics that characterize various behaviors of human, and promotion thereof is often caused by central neurological diseases, namely, mental diseases, neurodegenerative diseases and the like, and strongly associated with violence, aggressive behavior, suicide and the like. According to the biopsychosocial definition of impulsivity, impulsivity is defined as a predisposition toward rapid, unplanned reactions to internal or external stimuli without regard to the negative consequences of these reactions to the impulsive individual or to others (Am J Psychiatry 2001; 158:1783-93). Moreover, Barratt Impulsiveness Scale can evaluate the properties of impulsivity a person has, based on three subscales of impulsivity caused by attention ability, behavioral impulsivity, impulsivity due to lack of plan and the like (J Clin Psychol 1995; 51:768-74).

Examples of the mental diseases with impulsive symptoms include schizophrenia, major depression, bipolar disorder, attention deficit hyperactivity disorder (AD/HD), autism, antisocial personality disorder, borderline personality disorder, substance abuse/dependence and the like.

While many of schizophrenia patients do not show violent behaviors, a part of the patients shows sustained aggressive behavior, and sometimes prevent medication or place a burden on caregivers. In the longitudinal epidemiologic study conducted in Sweden in 1973 - 2006, 5.3% of the population was involved in crime of violence; however, 13.2% of schizophrenia patients were involved in crime of violence (JAMA 2009; 301:2016-23). The cause of the violent behavior in schizophrenia patients has ununiformity, which is considered to derive from i) positive symptom such as hallucination-delusion and the like, ii) impulsivity, iii) concurrent psychopathy (Int J Clin Pract 2008; 62:1237-45).

Citrome et al. studied an aggression suppressive effect of existing antipsychotic agents on schizophrenia patients by a prospective randomized, double-blind trial using clozapine, olanzapine, risperidone, and haloperidol (Psychiatric Services 2001; 52:1510-14). For evaluation, hostility, which is one of the positive scales of PANSS (Positive and Negative Symptom Scale), was used. Clozapine solely attenuated statistically significant hostility and, on the other hand, risperidone and haloperidol aggravated hostility. Olanzapine only showed a minimum improving effect. Since clozapine shows a certain level of effect on the hostility in schizophrenia patients, its administration to schizophrenia patients showing violent behavior is recommended. However, clozapine is an antipsychotic agent having extremely strong efficacy, and the effect thereof could be suppression of violent behavior by the improvement of the above-mentioned i) positive symptom. It has not been verified whether the violent behavior deriving from ii) impulsivity could be suppressed. Moreover, since clozapine causes severe side effects such as agranulocytosis and the like, medical institutions and patients capable of using this drug are limited.

There are more than 100 reports on genes relating to the onset of schizophrenia, which are based on pedigree analysis, analysis of gene polymorphism and the like. Among those, the Disrupted-In-Schizophrenia 1 (Disc1) gene with reciprocal translocation of chromosome 1 and chromosome 11, which was found in schizophrenia multiplex families in Scotland, has been attracting attention as a weakness factor causing schizophrenia. The mouse having a Disc1 L100P point mutation showed schizophrenia-like abnormal behaviors and antipsychotic agents such as clozapine and haloperidol reduced the schizophrenia-like behaviors. Furthermore, a decrease in the brain volume and biochemical analyses have shown its usefulness as a schizophrenia-like model (Neuron 54(3): 387-402, 2007).

Major depression is strongly related to the suicide tendency, and impulsive symptoms are considered as important predictive factors thereof (Am J Psychiatry 1999; 156:181-89). Patients with major depression are more impulsive than healthy human (Am J Psychiatry 2005; 162:1680-7), and more prone to suicide attempt and suicidal act (Prog Neuropsychopharmacol Biol Psychiatry 2003; 27:829-33, Epidemiol Psichiatr Soc 2009; 18:172-8). The relation of selective serotonin re-uptake inhibitors (SSRI) used as antidepressants to an increase in the suicide risk was pointed out, and U.S. Food and Drug Administration (FDA) warned in 2004 that administration of an antidepressant may cause activation syndrome (AS) which may induce suicide. Harada et al. performed a retrospective search of AS emergence in outpatients prescribed with antidepressants for 3 months, and found that 4.3% of them showed AS (Depress Anxiety 2008; 25:1014-9). Therefore, when AS emerges after administration of an antidepressant in clinical situations, judgment of whether it is a side effect of the antidepressant or aggravation of the present illness is difficult, and the doctors often struggle to judge whether or not to continue administration of the antidepressant. Thus, the establishment of an appropriate therapy for major depression patients with high impulsive symptoms is desired.

As for violent behaviors in bipolar disorder, a report has been documented by Barlow et al. (Aust N Z J Psychiatry 2000; 34:967-74). A research of 1269 hospitalized patients with mental diseases over 18 months revealed that bipolar disorder patients in a manic state showed the highest odds ratio of violent behavior. In addition, the majority of violent behavior is considered to clearly stem from impulsivity, and many violent behaviors emerged with the manic episodes.

Clinically, mood stabilizers and antipsychotic agents are prescribed for the treatment of the manic episodes. While the effectiveness of the prescription has been reported by meta-analyses (Arch Gen Psychiatry 2007; 64:442-55, Acta Psychiatr Scand 2007; 115:12-20), a test studying the violent behavior does not exist at present.

Alcohol dependence and drug dependence are mental diseases satisfying several diagnostic criteria such as resistance, craving, withdrawal symptom and the like relating to alcohol or drug. It is well known that dependence patients take impulsive behaviors. Not only they cannot suppress an intake action of alcohol and drugs, but they take quick action to satisfy the immediate desire even though it can lead to an undesirable effect in the future. As such, the patients often commit a crime such as violent behavior and the like. It is said that such impulsive behavior is associated with a disorder in the prefrontal cortex (Pharmacol Biochem Behav 2009; 93:237-47). For the treatment of alcohol dependence, opioid antagonists such as naltrexone and nalmefene are prescribed to suppress impulsive alcohol drinking and help control alcohol intake. However, the treatment effect thereof is not sufficient, and the establishment of a medicament and a treatment method affording a higher treatment effect is desired.

Examples of the neurodegenerative disease include dementia [Alzheimer-type dementia (AD), dementia with Lewy bodies, Parkinson's type dementia, frontotemporal dementia, cerebrovascular dementia, Huntington's disease], multiple sclerosis and the like.

Examples of the behavioral and psychological symptoms in neurodegenerative diseases include behavioral and psychological symptoms of dementia and the like.

Dementia is divided into "core symptoms" mainly showing cognitive impairment such as memory, orientation, discernment and the like, and "behavioral and psychological symptoms" which are psychological symptoms and impulsive symptoms that appear in association with the "core symptoms". Psychological symptoms include depression, anxiety, hallucination, delusion, sleep disorder and the like, and impulsive symptoms include violence, violent language, wandering, rejection, unclean behavior and the like. In the International Psychogeriatric Association held in USA in 1995, these behavioral disturbances were defined as "symptoms of disturbed perception, thought content, mood or behavior that frequently occur in patients with dementia", and thereafter referred to as BPSD (Behavioral and Psychological Symptoms of Dementia).

According to epidemiologic researches, 80% of dementia patients at home show abnormality in behavior, i.e., BPSD, and BPSD emerges more often as the dementia progresses from the mild stage to the moderate stage. Since the home care gradually becomes difficult, QOL (Quality of Life) of the patients and caregivers is degraded markedly. Comparatively mild BPSD sometimes can be dealt with by a "non-drug therapy" which includes appropriately improving living environment and care. However, when the stage is moderate or above and various problems have occurred such as increased stress of not only patients but also caregivers, and the like, a drug treatment is necessary in many cases.

As for Alzheimer's disease which is a representative neurodegenerative disease, there is a report on the study of a treatment effect of donepezil for 12 weeks on agitation, which is one of the behavioral and psychological symptoms (N Engl J Med 2007, 357:1382-1392). Patients with highly severe Alzheimer's disease, who were cared for in a nursing home, were divided into a placebo group and a donepezil administration group, and a test was performed. They were evaluated by CMAI (Cohen-Mansfield Agitation Inventory) and NPI (Neuropsychiatric Inventory). As a result, each score showed no statistically significant difference between the placebo group and the donepezil administration group, and the score itself showed almost no change (p value: CMAI 0.98, NPI 0.95). The results can be interpreted to mean that donepezil did not improve or promote agitation. Therefore, donepezil is a medicament expected to improve cognitive function in Alzheimer's disease, but shows no improving effect on behavioral and psychological symptoms, particularly agitation, that often place a burden on the caregivers.

Alexander et al. reports on a BPSD model taking note of aggression, by using Tg2576 mouse, which is one of the AD model mice used worldwide (Behavioural Brain Research 2011; 216:77-83). Tg2576 is an AD model mouse having Swedish and London type Amyloid Precursor Protein (APP) mutations. In this model applying a "resident-intruder" test method, A/J mouse (intruder) of a lineage without aggressiveness is made to invade in a cage of individually-bred Tg2576 (resident). The aggressiveness of 7-month-old Tg2576 was studied. The time necessary for the first attack significantly decreased as compared to the control mouse, and the number of aggression for 10 min significantly increased.

In addition, Vloeberghs et al. reports on the change of the amount of the spontaneous locomotor activity based on the night circadian rhythm of APP23 mouse (Eur J Neurosci 2004; 10:2757-66). APP23 mouse is an AD model mouse having a Swedish APP mutation. 12-month-old APP23 and wild-type mice were compared for 3 days. As a result, the spontaneous locomotor activity of the wild-type mouse was high at night only the initial day, and significantly decreased on day 2 and day 3. On the other hand, APP23 mouse showed a high increase in the spontaneous locomotor activity for 3 nights. The spontaneous locomotor activity of APP23 mouse on days 2 and 3 at night significantly increased as compared to that of the wild-type on days 2 and 3.

As evidenced in the above-mentioned reports, there are a number of research reports of AD model mice showing partial BPSD symptoms, and the research and development of a therapeutic drug for BPSD is becoming possible using aggression and promoted spontaneous locomotor activity of these model mice as indices.

Dementia with Lewy bodies (DLB) is characterized by visual hallucination and auditory hallucination, and both of the progressive cognitive impairment and the Parkinson's disease-like movement disorder emerge as symptoms. Among senile degenerative dementing disorders, it is the second frequent next to Alzheimer-type dementia. DLB is a dementia most often accompanying BPSD from the early stages, and therefore, the QOL of the patients and caregivers is markedly impaired. Fujita et al. took note of the genetic mutation found in familial DLB, and succeeded in generating a novel transgenic mouse model expressing mutant P123Hβ-synuclein (Nat Commun 2010; 1:110). This mouse shows cognitive symptoms in addition to various pathological findings, and further shows BPSD-like abnormal behaviors. Therefore, the research and development of a therapeutic drug for BPSD is also possible by using this model mouse.

As mentioned above, once behavioral and psychological symptoms associated with neurodegenerative disease or impulsive symptoms associated with mental disease are developed, a very heavy burden is placed on the caregivers and people around may be injured. Therefore, a medicament that suppresses such symptoms is desired.

### Summary of the Invention

An object of the present invention is to provide a prophylactic and/or therapeutic agent which is superior in safety for behavioral and psychological symptoms associated with neurodegenerative disease or impulsive symptoms associated with mental disease.

In an attempt to solve the aforementioned problems, the present inventors have conducted intensive studies using aggression and promoted spontaneous locomotor activity and the like of AD model mouse having an APP genetic mutation as indices and found that brexpiprazole or a salt thereof is effective for the behavioral and psychological symptoms associated with neurodegenerative disease or impulsive symptoms associated with mental disease. Furthermore, they have found that brexpiprazole or a salt thereof activates nerve cell of the medial prefrontal cortex deeply related to the behavioral and psychological symptoms associated with neurodegenerative disease and impulsive symptoms of mental disease.

The present invention provides a prophylactic and/or therapeutic agent for behavioral and psychological symptoms associated with neurodegenerative disease or impulsive symptoms associated with mental disease, which contains brexpiprazole or a salt thereof as an active ingredient.

The present invention provides a composition (pharmaceutical composition) for the prophylaxis and/or treatment of behavioral and psychological symptoms associated with neurodegenerative disease or impulsive symptoms associated with mental disease, which contains brexpiprazole or a salt thereof as an active ingredient.

The present invention provides use of brexpiprazole or a salt thereof for producing a prophylactic and/or therapeutic agent for behavioral and psychological symptoms associated with neurodegenerative disease or impulsive symptoms associated with mental disease.

The present invention provides a method for the prophylaxis and/or treatment of behavioral and psychological symptoms associated with neurodegenerative disease or impulsive symptoms associated with mental disease, which comprises administering brexpiprazole or a salt thereof in a prophylactically or therapeutically effective amount to a patient in need of the prophylaxis and/or treatment of behavioral and psychological symptoms associated with neurodegenerative disease or impulsive symptoms associated with mental disease.

The present invention provides a method for the prophylaxis and/or treatment of behavioral and psychological symptoms associated with neurodegenerative disease or impulsive symptoms associated with mental disease, which comprises administering brexpiprazole or a salt thereof in a prophylactically or therapeutically effective amount to a patient for whom generally available antipsychotic agents or therapeutic drugs for neurodegenerative disease fail to provide a sufficient effect, from among the patients in need of the prophylaxis and/or treatment of behavioral and psychological symptoms associated with neurodegenerative disease or impulsive symptoms associated with mental disease.

That is, the present invention provides prophylactic and/or therapeutic agents for behavioral and psychological symptoms associated with central neurological disease shown in the following Items 1 to 59.

### Item 1.

A method for the prophylaxis and/or treatment of behavioral and psychological symptoms associated with neurodegenerative disease or impulsive symptoms associated with mental disease, which comprises administering 7-[4-(4-benzo[b]thiophen-4-yl-piperazin-1-yl)butoxy]-1H-quinolin-2-one or a salt thereof in a prophylactically or therapeutically effective amount to a patient in need of the prophylaxis and/or treatment of behavioral and psychological symptoms associated with neurodegenerative disease or impulsive symptoms associated with mental disease.

### Item 2.

The method for the prophylaxis and/or treatment of Item 1, which is a method for the prophylaxis and/or treatment of behavioral and psychological symptoms associated with neurodegenerative disease.

### Item 3.

The method for the prophylaxis and/or treatment of Item 1, which is a method for the prophylaxis and/or treatment of impulsive symptoms associated with mental disease.

### Item 4.

The method for the prophylaxis and/or treatment of Item 2, wherein the neurodegenerative disease is selected from the group consisting of dementia, multiple sclerosis, Parkinson syndrome, juvenile parkinsonism, striatonigral degeneration, progressive supranuclear palsy, pure akinesia, prion disease, corticobasal degeneration, chorea-acanthocytosis, benign hereditary chorea, paroxysmal choreoathetosis, essential tremor, essential myoclonus, Gilles de la Tourette syndrome, Rett syndrome, degenerative ballism, dystonia musculorum deformans, athetosis, spasmodic torticollis, Meige syndrome, cerebral palsy, Wilson's disease, Segawa's disease, Hallervorden-Spatz syndrome, neuroaxonal dystrophy, pallidal atrophy, spinocerebellar degeneration, cerebral cortical atrophy, Holmes-type cerebellar atrophy, olivopontocerebellar atrophy, hereditary olivopontocerebellar atrophy, Joseph disease, dentatorubropallidoluysian atrophy, Gerstmann-Straussler-Scheinker syndrome, Friedreich ataxia, Roussy-Levy syndrome, May-White syndrome, congenital cerebellar ataxia, periodic hereditary ataxia, ataxia telangiectasia, amyotrophic lateral sclerosis, progressive bulbar palsy, spinal progressive muscular atrophy, spinobulbar muscular atrophy, Werdnig-Hoffmann disease, Kugelberg-Welander disease, hereditary spastic paraplegia, syringomyelia, syringobulbia, Arnold-Chiari malformation, stiff man syndrome, Klippel-Feil syndrome, Fazio-Londe disease, low myelopathy, Dandy-Walker syndrome, spina bifida, Sjogren-Larsson syndrome, radiation myelopathy, age-related macular degeneration, and cerebral apoplexy due to cerebral hemorrhage and/or dysfunction or neurologic deficits associated therewith.

### Item 5.

The method for the prophylaxis and/or treatment of Item 4, wherein the neurodegenerative disease is dementia.

### Item 6.

The method for the prophylaxis and/or treatment of Item 5, wherein the dementia is Alzheimer-type dementia.

### Item 7.

The method for the prophylaxis and/or treatment of Item 5, wherein the dementia is dementia with Lewy bodies.

### Item 8.

The method for the prophylaxis and/or treatment of Item 5, wherein the dementia is frontotemporal dementia.

### Item 9.

The method for the prophylaxis and/or treatment of Item 5, wherein the dementia is cerebrovascular dementia.

### Item 10.

The method for the prophylaxis and/or treatment of Item 5, wherein the dementia is Parkinson's type dementia.

### Item 11.

The method for the prophylaxis and/or treatment of Item 5, wherein the dementia is Huntington's disease.

### Item 12.

The method for the prophylaxis and/or treatment of Item 4, wherein the neurodegenerative disease is multiple sclerosis.

### Item 13.

The method for the prophylaxis and/or treatment of Item 3, wherein the mental disease is selected from the group consisting of schizophrenia, treatment-resistant schizophrenia, refractory schizophrenia, chronic schizophrenia, emotional disturbance, psychotic disorder, mood disorder, bipolar disorder, mania, depression, endogenous depression, major depression, melancholic and treatment-resistant depression, dysthymic disorder, cyclothymic disorder, anxiety disorder, somatoform disorder, factitious disorder, dissociative disorder, sexual disorder, eating disorder, sleep disorder, adjustment disorder, substance-related disorder, anhedonia, delirium, vomiting, motion sickness, obesity, migraine, pain, mental retardation, autism, Tourette's disorder, tic disorder, attention deficit hyperactivity disorder, conduct disorder, intermittent explosive disorder, kleptomania, pyromania, pathological gambling, trichotillomania, Down's syndrome and personality disorder.

### Item 14.

The method for the prophylaxis and/or treatment of Item 13, wherein the mental disease is selected from the group consisting of schizophrenia, treatment-resistant schizophrenia, refractory schizophrenia and chronic schizophrenia.

### Item 15.

The method for the prophylaxis and/or treatment of Item 13, wherein the mental disease is selected from the group consisting of depression, endogenous depression, major depression, melancholic and treatment-resistant depression.

### Item 16.

The method for the prophylaxis and/or treatment of Item 13, wherein the mental disease is bipolar disorder.

### Item 17.

The method for the prophylaxis and/or treatment of Item 13, wherein the mental disease is eating disorder.

### Item 18.

The method for the prophylaxis and/or treatment of Item 13, wherein the mental disease is attention deficit hyperactivity disorder.

### Item 19.

The method for the prophylaxis and/or treatment of Item 13, wherein the mental disease is anxiety disorder.

### Item 20.

The method for the prophylaxis and/or treatment of Item 19, wherein the anxiety disorder is obsessive-compulsive disorder.

### Item 21.

The method for the prophylaxis and/or treatment of Item 19, wherein the anxiety disorder is post-traumatic stress disorder.

### Item 22.

The method for the prophylaxis and/or treatment of any one of Items 1 to 21, wherein the patient cannot receive a sufficient effect for behavioral and psychological symptoms associated with neurodegenerative disease or impulsive symptoms associated with mental disease from a generally available antipsychotic agent or therapeutic drug for neurodegenerative disease.

### Item 23.

The method for the prophylaxis and/or treatment of Item 22, wherein the generally available antipsychotic agent is chlorpromazine, fluphenazine, levomepromazine, perphenazine, propericiazine, bromperidol, haloperidol, pipamperone, timiperone, nemonapride, sulpiride, sultopride, carpipramine, clocapramine, mosapramine, pimozide, oxypertine, zotepine, amisulpride, risperidone, iloperidone, perospirone, paliperidone, lurasidone, ziprasidone, asenapine, clozapine, olanzapine, quetiapine, blonanserin, aripiprazole, cariprazine or sertindole, or a salt thereof.

### Item 24.

The method for the prophylaxis and/or treatment of Item 22, wherein the generally available therapeutic drug for neurodegenerative disease is donepezil, galantamine, rivastigmine, memantine, fingolimod, methylprednisolone, azathioprine, mitoxantrone, cyclophosphamide, interferon β preparation, glatiramer, teriflunomide or natalizumab, or a salt thereof.

### Item 25.

A prophylactic and/or therapeutic agent for behavioral and psychological symptoms associated with neurodegenerative disease or impulsive symptoms associated with mental disease, comprising 7-[4-(4-benzo[b]thiophen-4-yl-piperazin-1-yl)butoxy]-1H-quinolin-2-one or a salt thereof as an active ingredient.

### Item 26.

The prophylactic and/or therapeutic agent of Item 25, which is a prophylactic and/or therapeutic agent for behavioral and psychological symptoms associated with neurodegenerative disease.

### Item 27.

The prophylactic and/or therapeutic agent of Item 25, which is a prophylactic and/or therapeutic agent for impulsive symptoms associated with mental disease.

### Item 28.

The prophylactic and/or therapeutic agent of Item 26, wherein the neurodegenerative disease is selected from the group consisting of dementia, multiple sclerosis, Parkinson syndrome, juvenile parkinsonism, striatonigral degeneration, progressive supranuclear palsy, pure akinesia, prion disease, corticobasal degeneration, chorea-acanthocytosis, benign hereditary chorea, paroxysmal choreoathetosis, essential tremor, essential myoclonus, Gilles de la Tourette syndrome, Rett syndrome, degenerative ballism, dystonia musculorum deformans, athetosis, spasmodic torticollis, Meige syndrome, cerebral palsy, Wilson's disease, Segawa's disease, Hallervorden-Spatz syndrome, neuroaxonal dystrophy, pallidal atrophy, spinocerebellar degeneration, cerebral cortical atrophy, Holmes-type cerebellar atrophy, olivopontocerebellar atrophy, hereditary olivopontocerebellar atrophy, Joseph disease, dentatorubropallidoluysian atrophy, Gerstmann-Straussler-Scheinker syndrome, Friedreich ataxia, Roussy-Levy syndrome, May-White syndrome, congenital cerebellar ataxia, periodic hereditary ataxia, ataxia telangiectasia, amyotrophic lateral sclerosis, progressive bulbar palsy, spinal progressive muscular atrophy, spinobulbar muscular atrophy, Werdnig-Hoffmann disease, Kugelberg-Welander disease, hereditary spastic paraplegia, syringomyelia, syringobulbia, Arnold-Chiari malformation, stiff man syndrome, Klippel-Feil syndrome, Fazio-Londe disease, low myelopathy, Dandy-Walker syndrome, spina bifida, Sjogren-Larsson syndrome, radiation myelopathy, age-related macular degeneration, and cerebral apoplexy due to cerebral hemorrhage and/or dysfunction or neurologic deficits associated therewith.

### Item 29.

The prophylactic and/or therapeutic agent of Item 28, wherein the neurodegenerative disease is dementia.

### Item 30.

The prophylactic and/or therapeutic agent of Item 29, wherein the dementia is Alzheimer-type dementia.

### Item 31.

The prophylactic and/or therapeutic agent of Item 29, wherein the dementia is dementia with Lewy bodies.

### Item 32.

The prophylactic and/or therapeutic agent of Item 29, wherein the dementia is frontotemporal dementia.

### Item 33.

The prophylactic and/or therapeutic agent of Item 29, wherein the dementia is cerebrovascular dementia.

### Item 34.

The prophylactic and/or therapeutic agent of Item 29, wherein the dementia is Parkinson's type dementia.

### Item 35.

The prophylactic and/or therapeutic agent of Item 29, wherein the dementia is Huntington's disease.

### Item 36.

The prophylactic and/or therapeutic agent of Item 28, wherein the neurodegenerative disease is multiple sclerosis.

### Item 37.

The prophylactic and/or therapeutic agent of Item 27, wherein the mental disease is selected from the group consisting of schizophrenia, treatment-resistant schizophrenia, refractory schizophrenia, chronic schizophrenia, emotional disturbance, psychotic disorder, mood disorder, bipolar disorder, mania, depression, endogenous depression, major depression, melancholic and treatment-resistant depression, dysthymic disorder, cyclothymic disorder, anxiety disorder, somatoform disorder, factitious disorder, dissociative disorder, sexual disorder, eating disorder, sleep disorder, adjustment disorder, substance-related disorder, anhedonia, delirium, vomiting, motion sickness, obesity, migraine, pain, mental retardation, autism, Tourette's disorder, tic disorder, attention deficit hyperactivity disorder, conduct disorder, intermittent explosive disorder, kleptomania, pyromania, pathological gambling, trichotillomania, Down's syndrome and personality disorder.

### Item 38.

The prophylactic and/or therapeutic agent of Item 37, wherein the mental disease is selected from the group consisting of schizophrenia, treatment-resistant schizophrenia, refractory schizophrenia and chronic schizophrenia.

### Item 39

The prophylactic and/or therapeutic agent of Item 37, wherein the mental disease is selected from the group consisting of depression, endogenous depression, major depression, melancholic and treatment-resistant depression.

### Item 40.

The prophylactic and/or therapeutic agent of Item 37, wherein the mental disease is bipolar disorder.

### Item 41.

The prophylactic and/or therapeutic agent of Item 37, wherein the mental disease is eating disorder.

### Item 42.

The prophylactic and/or therapeutic agent of Item 37, wherein the mental disease is attention deficit hyperactivity disorder.

### Item 43.

The prophylactic and/or therapeutic agent of Item 37, wherein the mental disease is anxiety disorder.

### Item 44.

The prophylactic and/or therapeutic agent of Item 43, wherein the anxiety disorder is obsessive-compulsive disorder.

### Item 45.

The prophylactic and/or therapeutic agent of Item 43, wherein the anxiety disorder is post-traumatic stress disorder.

### Item 46.

The prophylactic and/or therapeutic agent of any one of Items 25 to 45, for the treatment of a patient who cannot receive a sufficient effect for behavioral and psychological symptoms associated with neurodegenerative disease or impulsive symptoms associated with mental disease from a generally available antipsychotic agent or therapeutic drug for neurodegenerative disease.

### Item 47.

The prophylactic and/or therapeutic agent of Item 46, wherein the generally available antipsychotic agent is chlorpromazine, fluphenazine, levomepromazine, perphenazine, propericiazine, bromperidol, haloperidol, pipamperone, timiperone, nemonapride, sulpiride, sultopride, carpipramine, clocapramine, mosapramine, pimozide, oxypertine, zotepine, amisulpride, risperidone, iloperidone, perospirone, paliperidone, lurasidone, ziprasidone, asenapine, clozapine, olanzapine, quetiapine, blonanserin, aripiprazole, cariprazine or sertindole, or a salt thereof.

### Item 48.

The prophylactic and/or therapeutic agent of Item 46, wherein the generally available therapeutic drug for neurodegenerative disease is donepezil, galantamine, rivastigmine, memantine, fingolimod, methylprednisolone, azathioprine, mitoxantrone, cyclophosphamide, interferon β preparation, glatiramer, teriflunomide or natalizumab, or a salt thereof.

### Item 49.

Use of 7-[4-(4-benzo[b]thiophen-4-yl-piperazin-1-yl)butoxy]-1H-quinolin-2-one or a salt thereof for producing a prophylactic and/or therapeutic agent for behavioral and psychological symptoms associated with neurodegenerative disease or impulsive symptoms associated with mental disease.

### Item 50.

7-[4-(4-Benzo[b]thiophen-4-yl-piperazin-1-yl)butoxy]-1H-quinolin-2-one or a salt thereof for use in the prophylaxis and/or treatment of behavioral and psychological symptoms associated with neurodegenerative disease or impulsive symptoms associated with mental disease.

### Item 51.

A pharmaceutical composition for use in the prophylaxis and/or treatment of behavioral and psychological symptoms associated with neurodegenerative disease or impulsive symptoms associated with mental disease, which comprises 7-[4-(4-benzo[b]thiophen-4-yl-piperazin-1-yl)butoxy]-1H-quinolin-2-one or a salt thereof as an active ingredient.

### Item 52.

The method for the prophylaxis and/or treatment of Item 13, wherein the mental disease is substance-related disorder.

### Item 53.

The method for the prophylaxis and/or treatment of Item 52, wherein the substance-related disorder is alcohol-related disorder.

### Item 54.

The prophylactic and/or therapeutic agent of Item 37, wherein the mental disease is substance-related disorder.

### Item 55.

The prophylactic and/or therapeutic agent of Item 54, wherein the substance-related disorder is alcohol-related disorder.

### Item 56.

The method for the prophylaxis and/or treatment of Item 6, wherein the behavioral and psychological symptoms are impulsive symptoms.

### Item 57.

The method for the prophylaxis and/or treatment of Item 56, wherein the impulsive symptom is agitation.

### Item 58.

The prophylactic and/or therapeutic agent of Item 30, wherein the behavioral and psychological symptoms are impulsive symptoms.

### Item 59.

The prophylactic and/or therapeutic agent of Item 58, wherein the impulsive symptom is agitation.

### Effect of the Invention

Brexpiprazole or a salt thereof has a superior treatment effect for behavioral and psychological symptoms associated with neurodegenerative disease or impulsive symptoms associated with mental disease. Brexpiprazole or a salt thereof has a superior treatment effect particularly for behavioral and psychological symptoms associated with dementia (BPSD) (preferably Alzheimer's disease). It is also possible to improve those symptoms by additionally administering brexpiprazole or a salt thereof with an existing antipsychotic agent or therapeutic drug for neurodegenerative disease to a patient who cannot receive a sufficient effect from the existing drugs. Moreover, brexpiprazole or a salt thereof activates nerve cells in the medial prefrontal cortex. Furthermore, brexpiprazole or a salt thereof is superior to existing antipsychotic agents in the safety and tolerance, and can be safely administered to elderly Alzheimer's disease patients.

### Brief Description of the Drawings

Fig. 1 shows the results of a preliminary test confirming the promoted aggression of Tg2576 mouse.
Fig. 2 shows the test results of a suppressive effect of brexpiprazole on the aggression of Tg2576 mouse.
Fig. 3 shows the influence of the administration of brexpiprazole on the individual average ethanol intake in limited access paradigm.
Fig. 4 shows the effect of brexpiprazole on medial prefrontal cortex nerve activation pattern of c-fos-GFP mouse, wherein the area with a significant increase in the GFP signal relative to the vehicle group is shown white.

### Description of Embodiments

The active ingredient in the present invention is brexpiprazole or a salt thereof. Brexpiprazole is a known compound represented by the following formula and is under clinical tests for schizophrenia and the like.

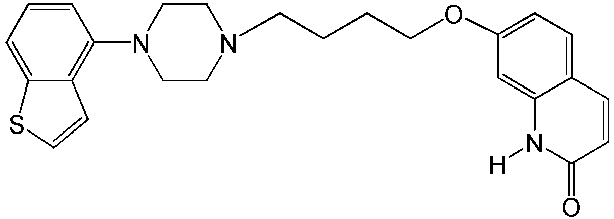

The salt of brexpiprazole is not particularly limited as long as it is a pharmacologically acceptable salt and, for example, metal salts such as alkali metal salts (e.g., sodium salt, potassium salt etc.), alkaline earth metal salts (e.g., calcium salt, magnesium salt etc.) and the like, ammonium salt, salts with inorganic base such as alkali metal carbonates (e.g., lithium carbonate, potassium carbonate, sodium carbonate, cesium carbonate etc.), alkali metal hydrogen carbonates (e.g., lithium hydrogen carbonate, sodium hydrogen carbonate, potassium hydrogen carbonate etc.), alkali metal hydroxides (e.g., lithium hydroxide, sodium hydroxide, potassium hydroxide, cesium hydroxide etc.) and the like; salts with organic base such as tri(lower)alkylamines (e.g., trimethylamine, triethylamine, N-ethyldiisopropylamine etc.), pyridine, quinoline, piperidine, imidazole, picoline, dimethylaminopyridine, dimethylaniline, N-(lower)alkylmorpholines (e.g., N-methylmorpholine etc.), 1,5-diazabicyclo[4.3.0]nonene-5 (DBN), 1,8-diazabicyclo[5.4.0]undecene-7 (DBU), 1,4-diazabicyclo[2.2.2]octane (DABCO) and the like; salts with inorganic acid such as hydrochloride, hydrobromide, hydroiodide, sulfate, nitrate, phosphate and the like; salts with organic acid such as formate, acetate, propionate, oxalate, malonate, succinate, fumarate, maleate, lactate, malate, citrate, tartrate, carbonate, picrate, methanesulfonate, ethanesulfonate, p-toluenesulfonate, glutamate and the like; and the like can be mentioned. As used herein, "(lower)alkyl" means "alkyl having 1 to 6 carbon atoms".

The "brexpiprazole or a salt thereof" includes anhydride and solvates (e.g., hydrate, preferably dihydrate) of brexpiprazole or a salt thereof, various crystal forms of these anhydride and solvates, and mixtures thereof.

One kind alone of such brexpiprazole or a salt thereof may be used, or a mixture of two or more kinds thereof may be used. anhydride of brexpiprazole or a salt thereof can be obtained by the methods described in, for example, Example 1 and Examples 42 to 47 of patent document 1 (JP-A-2006-316052 (US 2010/0179322 A1)).

Brexpiprazole or a salt thereof may be used in bulk or preferably in the form of a pharmaceutical preparation with a conventional pharmaceutical carrier (pharmaceutically acceptable carrier) or a diluent. The dosage form is not limited to a particular form. Specifically, it may be any conventional administration form, for example, an oral solid dosage form such as tablet, capsule and particles; various liquid preparations suitable for oral administration; or a parenteral preparation such as injection and suppository. The dose is not limited to a specific range. Generally, the active ingredient may be used in an amount of about 0.01 to 10 mg/day/1 kg of body weight. The active ingredient may be included in about 0.1-400 mg per a dosage unit of the preparation.

The preparation for injection is usually prepared in the form of a liquid preparation, an emulsion, or a suspension, which are sterilized and further are preferably made isotonic to the blood. The preparations in the form of liquid, emulsion or suspension are usually prepared by using conventional pharmaceutical diluents such as water, ethyl alcohol, propylene glycol, ethoxylated isostearyl alcohol, polyoxylated isostearyl alcohol, and polyoxyethylene sorbitan fatty acid esters. These preparations may be prepared by mixing with an isotonic agent such as sodium chloride, glucose, glycerol in an amount sufficient for making isotonic and may further be prepared by mixing with conventional solubilizers, buffers, anesthetizing agents, and optionally colorants, preservatives, fragrance substances, flavors or sweetening agents.

The preparations such as tablets, capsules, liquid for oral administration may be prepared by a conventional method. The tablets may be prepared by mixing brexpiprazole or a salt thereof with conventional pharmaceutical carriers such as gelatin, starches, lactose, magnesium stearate, talc, gum arabic, and the like. The capsules may be prepared by mixing brexpiprazole or a salt thereof with inert pharmaceutical fillers or diluents and filling hard gelatin capsules or soft capsules with the mixture. The oral liquid preparations such as syrups or elixirs are prepared by mixing brexpiprazole or a salt thereof with sweetening agents (e.g. sucrose), preservatives (e.g. methylparaben, propylparaben), colorants, flavors, and the like. The preparations for parenteral administration may also be prepared by a conventional method, for example, by dissolving brexpiprazole or a salt thereof in a sterilized aqueous carrier, preferably water or a saline solution. Preferred liquid preparation suitable for parenteral administration is prepared by dissolving about 0.1-400 mg of brexpiprazole or a salt thereof in water and an organic solvent and further in a polyethylene glycol having a molecular weight of 300 to 5000, in which preferably a lubricant such as sodium carboxymethylcellulose, methylcellulose, polyvinylpyrrolidone, and polyvinyl alcohol may be incorporated. Preferably, the above liquid preparations may further comprise a disinfectant (e.g. benzyl alcohol, phenol, thimerosal), an antimicrobial agent, and further optionally an isotonic agent (e.g. sucrose, sodium chloride), a topical anesthetic, a stabilizer, a buffer, and the like. In view of keeping stability, the preparation for parenteral administration may be put in a small container, followed by removing the aqueous medium by a conventional lyophilizing technique. The preparation can be recovered into a liquid preparation by dissolving it in an aqueous medium when used.

The present invention can prevent and/or treat behavioral and psychological symptoms associated with neurodegenerative disease or impulsive symptoms associated with mental disease by the administration of brexpiprazole or a salt thereof.

Examples of the mental disease in the present invention include schizophrenia, treatment-resistant schizophrenia, refractory schizophrenia, chronic schizophrenia, emotional disturbance, psychotic disorder, mood disorder, bipolar disorders (e.g., bipolar I disorder and bipolar II disorder and the like), mania, depression, endogenous depression, major depression, melancholic and treatment-resistant depression, dysthymic disorder, cyclothymic disorder, anxiety disorders (e.g., panic attack, panic disorder, agoraphobia, social phobia, obsessive-compulsive disorder, post-traumatic stress disorder, generalized anxiety disorder, acute stress disorder and the like), somatoform disorders (e.g., hysteria, somatization disorder, conversion disorder, pain disorder, hypochondria and the like), factitious disorder, dissociative disorder, sexual disorders (e.g., sexual dysfunction, sexual desire disorder, sexual arousal disorder, erectile dysfunction, paraphilias and the like), eating disorders (e.g., anorexia nervosa, bulimia nervosa and the like), sleep disorder, adjustment disorder, substance-related disorders (e.g., alcohol-related disorders (alcohol use disorder, alcohol-induced disorder, alcohol abuse, alcohol dependence, alcohol intoxication, alcohol withdrawal and the like), amphetamine-related disorders (amphetamine use disorder and the like), cannabis-related disorders (cannabis use disorder and the like), cocaine-related disorders (cocaine use disorder and the like), hallucinogen-related disorders (hallucinogen use disorder and the like) and the like), anhedonia (e.g., iatrogenic anhedonia, anhedonia of a psycic or mental cause, anhedonia associated with depression, anhedonia associated with schizophrenia and the like), delirium, vomiting, motion sickness, obesity, migraine, pain, mental retardation, autism, Tourette's disorder, tic disorder, attention deficit hyperactivity disorder, conduct disorder, Down's syndrome, personality disorder, intermittent explosive disorder, kleptomania, pyromania, pathological gambling, trichotillomania and the like.

Examples of the neurodegenerative disease in the present invention include dementia (e.g., Alzheimer-type dementia, dementia with Lewy bodies, frontotemporal dementia, cerebrovascular dementia, Parkinson's type dementia, Huntington's disease, senile dementia, mild cognitive impairment, HIV encephalopathy, corticobasal degeneration, Pick's disease, mixed dementia and the like), multiple sclerosis, Parkinson syndrome, juvenile parkinsonism, striatonigral degeneration, progressive supranuclear palsy, pure akinesia, prion disease, corticobasal degeneration, chorea-acanthocytosis, benign hereditary chorea, paroxysmal choreoathetosis, essential tremor, essential myoclonus, Gilles de la Tourette syndrome, Rett syndrome, degenerative ballism, dystonia musculorum deformans, athetosis, spasmodic torticollis, Meige syndrome, cerebral palsy, Wilson's disease, Segawa's disease, Hallervorden-Spatz syndrome, neuroaxonal dystrophy, pallidal atrophy, spinocerebellar degeneration, cerebral cortical atrophy, Holmes-type cerebellar atrophy, olivopontocerebellar atrophy, hereditary olivopontocerebellar atrophy, Joseph disease, dentatorubropallidoluysian atrophy, Gerstmann-Straussler-Scheinker syndrome, Friedreich ataxia, Roussy-Levy syndrome, May-White syndrome, congenital cerebellar ataxia, periodic hereditary ataxia, ataxia telangiectasia, amyotrophic lateral sclerosis, progressive bulbar palsy, spinal progressive muscular atrophy, spinobulbar muscular atrophy, Werdnig-Hoffmann disease, Kugelberg-Welander disease, hereditary spastic paraplegia, syringomyelia, syringobulbia, Arnold-Chiari malformation, stiff man syndrome, Klippel-Feil syndrome, Fazio-Londe disease, low myelopathy, Dandy-Walker syndrome, spina bifida, Sjogren-Larsson syndrome, radiation myelopathy, age-related macular degeneration, and cerebral apoplexy due to cerebral hemorrhage and/or associated dysfunctions or neurologic deficits and the like.

The behavioral and psychological symptoms in the present invention are impulsive symptoms and psychological symptoms.

The impulsive symptom is a symptom of taking an impulsive behavior. Specific examples of the impulsive behavior include physical attack, wandering, restlessness, agitation, senseless behavior and deviant behavior (e.g., sexual deviant behavior), roaming, shrill voice, screaming, violent language, loss of motivation, constant questioning, shadowing, suicide attempt and suicide, self-injurious behavior, threat, stealing, overeating, act of threatening, short-circuit reaction, panic reaction, property damage, inappropriate dressing/undressing, underselling and the like. In the preferred embodiment, the impulsive symptom is agitation.

Examples of the psychological symptom include hallucination, delusion, depressed mood, sleeplessness, anxiety, misrecognition, sleep disorder and the like.

The method for the prophylaxis and/or treatment of behavioral and psychological symptoms in the present invention means a method of preventing and/or treating a condition with manifestation of one or plural symptoms of the above-mentioned behavioral and psychological symptoms.

The method for the prophylaxis and/or treatment of impulsive symptoms in the present invention means a method of preventing and/or treating a condition with manifestation of one or plural symptoms of the above-mentioned impulsive symptoms.

Brexpiprazole or a salt thereof in the present invention is particularly useful for the prophylaxis and/or treatment of 1) behavioral and psychological symptoms associated with neurodegenerative disease, wherein the neurodegenerative disease is dementia (BPSD) (further, useful for the prophylaxis and/or treatment of, from among 1) behavioral and psychological symptoms associated with neurodegenerative disease wherein the neurodegenerative disease is dementia (BPSD), particularly, behavioral and psychological symptoms in association with Alzheimer-type dementia, behavioral and psychological symptoms in association with dementia with Lewy bodies, behavioral and psychological symptoms in association with frontotemporal dementia, behavioral and psychological symptoms in association with cerebrovascular dementia, behavioral and psychological symptoms in association with Parkinson's type dementia, behavioral and psychological symptoms in association with Huntington's disease), or 2) behavioral and psychological symptoms associated with neurodegenerative disease, wherein the neurodegenerative disease is multiple sclerosis.

Furthermore, brexpiprazole or a salt thereof in the present invention is particularly useful for the prophylaxis and/or treatment of, 1) impulsive symptoms associated with mental disease, wherein the mental disease is selected from the group consisting of schizophrenia, treatment-resistant schizophrenia, refractory schizophrenia, and chronic schizophrenia, 2) impulsive symptoms associated with mental disease, wherein the mental disease is selected from the group consisting of depression, endogenous depression, major depression, melancholic and treatment-resistant depression, 3) impulsive symptoms associated with mental disease, wherein the mental disease is bipolar disorder, 4) impulsive symptoms associated with mental disease, wherein the mental disease is eating disorder, 5) impulsive symptoms associated with mental disease, wherein the mental disease is attention deficit hyperactivity disorder, or 6) impulsive symptoms associated with mental disease, wherein the mental disease is anxiety disorder (further, useful for the prophylaxis and/or treatment of, from among 6) impulsive symptoms associated with mental disease, wherein the mental disease is anxiety disorder, impulsive symptoms associated with obsessive-compulsive disorder or post-traumatic stress disorder).

The above-mentioned symptom is not sometimes improved even in patients under medication with one or more kinds of antipsychotic agents and therapeutic drugs for neurodegenerative disease. The symptom can be improved by administering brexpiprazole or a salt thereof to such patients.

Examples of the existing (generally available) antipsychotic agent include chlorpromazine, fluphenazine, levomepromazine, perphenazine, propericiazine, bromperidol, haloperidol, pipamperone, timiperone, nemonapride, sulpiride, sultopride, carpipramine, clocapramine, mosapramine, pimozide, oxypertine, zotepine, amisulpride, risperidone, iloperidone, perospirone, paliperidone, lurasidone, ziprasidone, asenapine, clozapine, olanzapine, quetiapine, blonanserin, aripiprazole, cariprazine, sertindole or a salt thereof and the like.

Examples of the existing (generally available) therapeutic drug for neurodegenerative disease include Aricept (registered trade mark) (donepezil hydrochloride), Reminyl (registered trade mark) (galantamine hydrobromide), Exelon (registered trade mark) patch (rivastigmine transdermal absorption type preparation), Rivastach (registered trade mark) patch (rivastigmine transdermal absorption type preparation), Memary (registered trade mark) (memantine hydrochloride), fingolimod hydrochloride (Gilenya (registered trade mark) capsule, Imusera (registered trade mark) capsule), methylprednisolone, azathioprine, mitoxantrone, cyclophosphamide, interferon β preparation, Copaxone (registered trade mark) (glatiramer acetate), teriflunomide, Tysabri (registered trade mark) (natalizumab) and the like.

### Examples

### Example 1

### 1) Measurement of mouse circadian rhythm locomotor activity

Animal: APPSL-Tg mouse (male) having Swedish and London APP mutations, and non-Tg mouse (male) free of a genetic mutation as a control were generated (Neuroscience Letters 2010; 469:273-277), bred in a breeding room, and used after they became 6-month-old. During the breeding, isolated housing was applied.

Measurement method: SUPERMEX manufactured by Muromachi Kikai Co., Ltd. was used for the measurement of circadian rhythm locomotor activity. The mouse was placed in an individual cage, and the spontaneous locomotor activity of the mouse was measured for 3 days (total 62.5 hr) under free-feeding, drinking water conditions. In this apparatus, a passive infrared sensor detects infrared rays emitted from the mouse, and the number of transpositions is counted. The measured values are totaled every 30 min, and automatically totaled using a specialized software CompACT AMS. The test was performed in a soundproof chamber, so that the spontaneous locomotor activity of the mouse would not be influenced. The lighting time in the soundproof chamber was set to 7:00-19:00 as in the breeding room.

### 2) Grouping by preliminary test

The amounts of spontaneous locomotor activity of non-Tg mice and APPSL-Tg mice during the dark period of 19:00-7:00 were measured in advance. The mice were grouped in such manner as makes the mean and variance of the groups equal, using the body weight and the dark period spontaneous locomotor activity as indices.

### 3) Preparation of drug and administration method

Brexpiprazole was dissolved in distilled water containing 5% gum arabic, 5% gum arabic distilled water was used for the vehicle group, and they were orally administered to each mouse.

### 4) Number of mice and dose setting

group 1: 5 non-Tg mice/vehicle
group 2: 5 APPSL-Tg mice/vehicle
group 3: 6 APPSL-Tg mice/0.01 mg/kg brexpiprazole
group 4: 5 APPSL-Tg mice/0.03 mg/kg brexpiprazole

### 5) Administration time

For 3 days, brexpiprazole and the vehicle were administered during the period of 17:30-18:30. After the administration, the measurement of the amount of locomotor activity was rapidly started or continued.

### 6) Statistical analysis

The statistical test was a two-sided test, and the significance level of the test was set to 5%. As a statistical software, SAS (R9.1, SAS Institute Japan Ltd.) was used.

### i) Comparison of non-Tg mouse/vehicle group and APPSL-Tg mouse/vehicle group

A repeated measures ANOVA was performed using a mixed model for Night 1 - Night 3 at every phase I, II or III of the dark period. Furthermore, an unpaired t-test was performed for every dark period and Night.

### ii) Comparison of APPSL-Tg mouse/vehicle group and APPSL-Tg mouse/brexpiprazole administration group

Dunnett's test was performed based on the repeated measures ANOVA using a mixed model for Night 1 - Night 3 at every phase I, II or III of the dark period. Furthermore, Dunnett's test was performed for every dark period and Night.

### 7) Results

The test results are shown in Table 1 and Table 2.

**[Table 1]**

| Comparison of non-Tg mouse/vehicle group and APPSL-Tg mouse/vehicle group | | | | | | | |
|---|---|---|---|---|---|---|---|
| | Non-Tg/vehicle group (n=5) | | | APPSL-Tg/vehicle group (n=5) | | | |
| dark period | Night 1 | Night 2 | Night 3 | Night 1 | Night 2 | Night 3 | P value |
| I(19:00-23:00) | 6204 ± 2596 | 6489 ± 2309 | 7260 ± 1524 | 9377 ± 2395 | 11459 ± 3053 | 12839 ± 1960 | 0.1804 |
| II (23:00-3:00) | 5155 ± 1561 | 4678 ± 1199 | 3398 ± 518 | 8162 ± 1277 | 9438 ± 2146 | 13249 ± 3102* | 0.0250 |
| III(3:00-7: 00) | 2534 ± 597 | 2513 ± 765 | 3146 ± 616 | 9186 ± 955** | 7469 ± 572** | 10694 ± 1329** | 0.0001 |

| | | | | | | | |
|---|---|---|---|---|---|---|---|
| mean ± standard error P values show the results of the repeated measures ANOVA using mixed model. Furthermore, analyzed by an unpaired t-test for every Night in each dark period (*P<0.05; **P<0.01 vs Non-Tg/vehicle group). | | | | | | | |

**[Table 2]**

| Comparison of APPSL-Tg mouse/vehicle group and APPSL-Tg mouse/brexpiprazole administration group | | | | | | | |
|---|---|---|---|---|---|---|---|
| | APPSL-Tg/vehicle group (n=5) | | | APPSL-Tg/0.01 mg/kg brexpiprazole group (n=6) | | | |
| dark period | Night 1 | Night 2 | Night 3 | Night 1 | Night 2 | Night 3 | P value |
| III(3:00-7: 00) | 9186 ± 955 | 7469 ± 572 | 10694 ± 1329 | 7256 ± 774 | 6466 ± 707 | 4928 ± 483** | 0.049 |

| | APPSL-Tg/0.03 mg/kg brexpiprazole group (n=5) | | | |
|---|---|---|---|---|
| dark period | Night 1 | Night 2 | Night 3 | P value |
| III(3:00-7: 00) | 6014 ± 1468 | 5443 ± 1126 | 6845 ± 1554# | 0.050 |

| | | | | |
|---|---|---|---|---|
| mean ± standard error P values show the results of Dunnett's test based on the repeated measures ANOVA using mixed model. Furthermore, analyzed by Dunnett's test for every Night (**P<0.01, #P=0.068 vs APPSL-Tg/vehicle group). | | | | |

Heretofore, Vloeberghs et al. has reported, regarding the spontaneous locomotor activity of APP-Tg mouse model in 12 hr/12 hr light-dark cycle, that the dark period spontaneous locomotor activity is promoted as the aging proceeds (Eur J Neurosci. 2004; 10:2757-66). The APPSL-Tg mice used in the present invention also showed significantly increased spontaneous locomotor activity in phase II and phase III as compared to the Non-Tg group (phase II: P<0.05; phase III: P<0.01). Furthermore, for every Night in each dark period, the spontaneous locomotor activity increased significantly at Night 3, dark period phase II (P<0.05) or at Night 1 - Night 3, phase III (P<0.01) (Table 1).

Brexpiprazole was administered to APPSL-Tg mouse immediately before the dark period (17:30-18:30) at a dose of 0.01 and 0.03 mg/kg, and the measurement of the spontaneous locomotor activity was started. Brexpiprazole was administered at the equal time on Day 2 and Day 3, and the measurement of the spontaneous locomotor activity was continued. As a result, the 0.01 mg/kg group and 0.03 mg/kg group significantly suppress the spontaneous locomotor activity in dark period phase III as compared to the vehicle group (0.01 mg/kg group: P<0.05; 0.03 mg/kg group: P=0.050). Furthermore, for every Night in dark period phase III, 0.01 mg/kg brexpiprazole significantly suppressed the spontaneous locomotor activity at Night 3 (P<0.01, Table 2). Moreover, 0.03 mg/kg brexpiprazole also tended to suppress at Night 3 (P=0.068, Table 2). On the other hand, in non-Tg mouse, brexpiprazole did not decrease the spontaneous locomotor activity in dark period.

The above results have clarified that brexpiprazole can suppress, even at a low dose, abnormal behavior at night of AD model mouse having an APP genetic mutation.

### Example 2

### 1) Resident-Intruder test (impulsive symptoms study)

Animal: Tg2576 mice (male) having a Swedish APP mutation (K670N, M671L) and non-Tg mice (male) free of the same genetic mutation as a control were purchased from Taconic, and bred and aged until 5- to 6-month-old of age. During the breeding, isolated housing was applied.
Measurement method: For the experiment, Tg2576 or non-Tg mouse [Resident] and A/J mouse [Intruder] almost free of aggression were used. Resident formed a sufficient territory by isolated housing for 14 days. Thereafter, Intruder was moved to the Resident's cage, and the aggressive behavior was observed for 10 min. As the aggressive behavior, biting was noted, and the time necessary for the first biting and total number of biting for 10 min were measured. The measurement was performed within the dark period phase 1 (4 hr) when the amount of locomotor activity of the mouse is the highest.

### 2) Confirmation of aggression by preliminary test

Tg2576 (48 mice) and non-Tg mice (10 mice) were subjected to a Resident-Intruder test in advance, and promotion of the aggression of the Tg2576 mouse was confirmed. Five Tg2576 mice free of aggression were excluded and 43 mice were used for the test.

### 3) Grouping of Tg2576 mice and dose setting

The mice were grouped into 3 groups in such manner as makes the mean and variance of the groups equal, using the time necessary for the first attack and total number of biting for 10 min, which were obtained in the preliminary test, as indices (total 43 mice).
group 1: 15 Tg2576 mice/vehicle
group 2: 14 Tg2576 mice/0.01 mg/kg brexpiprazole (OPC-34712)
group 3: 14 Tg2576 mice/0.03 mg/kg brexpiprazole (OPC-34712)

### 4) Preparation of drug and administration method

Brexpiprazole was dissolved in distilled water containing 5% gum arabic, 5% gum arabic distilled water was used for the vehicle group, and they were orally administered to each mouse.

### 5) Administration time

Brexpiprazole and vehicle were administered 1 hr before the start of the test.

### 6) Statistical analysis

The significance level of the test was set to 5%. As a statistical software, SAS (R9.1, SAS Institute Japan Ltd.) was used. For confirmation of the promoted aggression of the Tg2576 mouse, analysis by a Wilcoxon rank sum test was performed as compared to the non-Tg mouse. As for an aggression suppressive effect by brexpiprazole administration, a Shirley-Williams' multiple comparison test was performed for analysis using the following combinations.
i) Tg2576 mouse/vehicle group and Tg2576 mouse/0.01 mg/kg brexpiprazole administration group
ii) Tg2576 mouse/vehicle group and Tg2576 mouse/0.03 mg/kg brexpiprazole administration group

### 7) Results

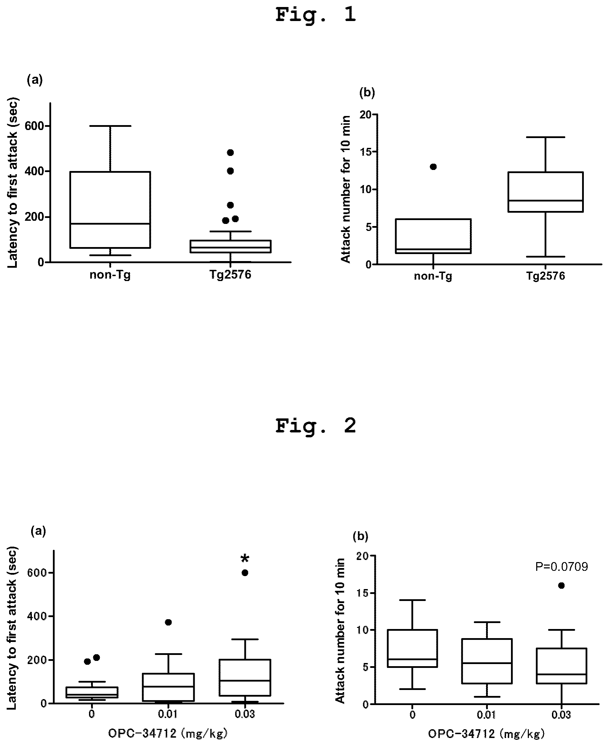

The test results are shown in Fig. 1 and Fig. 2.

Heretofore, Alexander et al. has reported, regarding the aggression of Tg2576 mouse, promotion of aggression, by using a Resident-Intruder test (Behavioural Brain Research 2011; 216:77-83). Tg2576 mouse used in the present invention was also evaluated by the Resident-Intruder test. As a result, the time necessary for the first biting was significantly shortened as compared to the Non-Tg group (Fig. 1a, P<0.05, Wilcoxon rank sum test). Furthermore, the total number of biting for 10 min was also analyzed. As a result, the Tg2576 mouse showed a significant increase in the number of biting (Fig. 1b, P<0.01, Wilcoxon rank sum test). In this manner, the aggression suppress effect of brexpiprazole was continuously studied using the same Tg2576 mouse showing clear promotion of aggressive behavior.

Brexpiprazole was administered to Tg2576 mouse at the doses of 0.01 and 0.03 mg/kg 1 hr before the start of the Resident-Intruder test, and the aggression suppressive effect of brexpiprazole was studied. Based on the measurement results, the time necessary for the first biting was analyzed. As a result, the time necessary for the first biting was significantly elongated in the 0.03 mg/kg group as compared to the vehicle group (Fig. 2a, vehicle group vs 0.03 mg/kg group: *P<0.05, Shirley-Williams' multiple comparison test). The number of biting was also analyzed by the same test. As a result, the Tg2576 mouse administered with 0.03 mg/kg brexpiprazole tended to show a decreased number of biting as compared to the vehicle group (Fig. 2b, vehicle group vs 0.03 mg/kg group: P=0.0709).

The above results have clarified that brexpiprazole can suppress aggression in the aggressive behavior of AD model mouse having an APP genetic mutation.

### Example 3

Suppression of behavioral and psychological symptoms by brexpiprazole can be evaluated by performing the measurement of circadian rhythm locomotor activity conducted in Example 1 and the Resident-Intruder test in Example 2, and general behavioral evaluation studies (elevated plus maze test, forced swimming test, tail suspension test, light/dark box test, marble burying behavior test, cliff avoidance response test), by using a novel transgenic mouse model that expresses mutant P123Hβ-synuclein.

### Example 4

Taking note of the impulsive symptoms of a mouse having a Disc1 L100P point mutation, suppression of impulsive symptoms by brexpiprazole can be evaluated by performing the measurement of circadian rhythm locomotor activity conducted in Example 1 and the Resident-Intruder test in Example 2 and general behavioral evaluation studies (elevated plus maze test, forced swimming test, tail suspension test, light/dark box test, marble burying behavior test, cliff avoidance response test).

### Example 5

Multicenter, randomized, double-blind, placebo-controlled study to examine treatment effect, safety, and tolerability of brexpiprazole (OPC-34712) in the treatment of subjects with agitation associated with dementia of the Alzheimer's type.

### Test method

55- to 90-year-old patients diagnosed with Alzheimer's disease according to National Institute of Neurological and Communicative Disorders and Stroke and the Alzheimer's Disease and Related Disorders Association (NINCDS-ADRDA) Alzheimer's Criteria, with a Mini Mental State Examination (MMSE) score of 5 to 22, and with a score of ≥ 4 on the agitation/aggression item of the Neuropsychiatric Inventory in Nursing Home Version (NPI-NH) were registered. The trial consists of a continuous 12-week double-blind treatment period. The subjects were assigned to one of the following groups.
- Placebo
- Brexpiprazole 0.5 mg (titrate up from 0.25 mg/day to 0.5 mg/day)
- Brexpiprazole 1 mg (titrate up from 0.25 mg/day to 1 mg/day)
- Brexpiprazole 2 mg (titrate up from 0.25 mg/day to 2 mg/day)

### Evaluation Method

The endpoint was evaluation of efficacy, safety, and tolerance of brexpiprazole by comparing the improvement of agitation associated with dementia of the Alzheimer's type between brexpiprazole groups and placebo group from recruiting patients to the final day of test period (week 12).

For evaluation of efficacy, the change in the Cohen-Mansfield Agitation Inventory (CMAI), the Clinical Global Impression of Severity (CGI-S) score, the CMAI subscale scores, the NPI-NH score (total, psychosis subscale, or individual item), the Clinical Global Impression-Improvement (CGI-I) score, and the Clinical Global Impression-Efficacy Index (CGI-E) score were used.

The suppression of behavioral and psychological symptoms associated with Alzheimer's disease by brexpiprazole, and safety and tolerability of brexpiprazole can be evaluated by performing the above.

### Example 6

### 1) Measurement of alcohol intake by limited access paradigm

Measurement method: An impulsive behavior of cravings for alcohol was evaluated as follows by reference to the method of Sinclair et al. (Alcohol 1992; 9:441-44 and Alcohol & Alcoholism 2001; 36:2-10). First, Wistar rat (male) was allowed to freely take 10% aqueous ethanol solution and tap water for several weeks under isolated housing. After stabilization of ethanol intake by each animal, a limited access paradigm allowing ethanol intake for only 1 hr per day was started, and the ethanol intake for 1 hr was measured every day. Ethanol intake was calculated from the results of the weight measurement of the water supply bottle filled with 10% aqueous ethanol solution immediately before the start of the limited access paradigm and immediately after completion thereof. An animal which was showing over 0.4 g/kg/hr in terms of 100% ethanol as an average ethanol intake in the limited access paradigm for 4 days immediately before drug evaluation was used. The limited access paradigm test was performed between 9:00 AM-12:00 PM.

### 2) Preparation of drug and administration method

Brexpiprazole was suspended in distilled water containing 5% gum arabic. The drug was orally administered to each rat once per day 1 hr before the start of the limited access paradigm for 4 days.

### 3) Number of animals and dose setting

Five rats were used. The selected dose of brexpiprazole was 0.1 mg/kg that does not influence spontaneous locomotor activity of Wistar rat (data not indicated) under novel environments.

### 4) Statistical analysis

The significance level of the test was set to 5%. As a statistical software, SAS (R9.3, SAS Institute Japan Ltd.) was used. An average ethanol intake in the limited access paradigm of 4 days immediately before drug evaluation and an average ethanol intake in the limited access paradigm of 4 days after drug administration were analyzed by the 2-tailed paired t-test.

### 5) Results

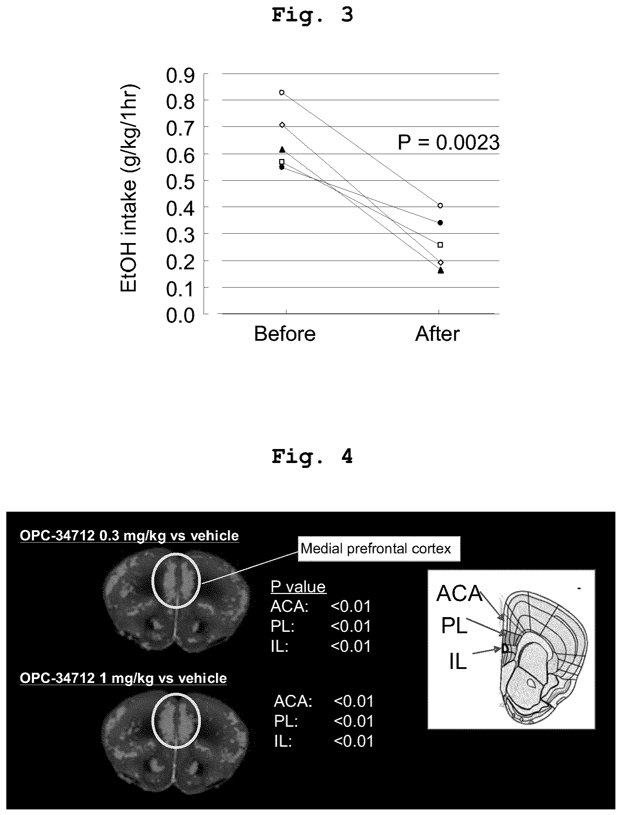

The test results are shown in Fig. 3.

A rat which was showing over 0.4 g/kg/hr as an average ethanol intake in the limited access paradigm for 4 days immediately before drug evaluation was administered with brexpiprazole one hour before at a dose of 0.1 mg/kg for 4 days, and average ethanol intake in the limited access paradigm was calculated. As a result, it was confirmed that brexpiprazole showed statistically significant suppression of ethanol intake. When observed individually, all rats showed a decrease in the ethanol intake.

The above results have clarified that brexpiprazole can suppress impulsive ethanol intake behavior of Wistar rat at a low dose in the limited access paradigm to 10% aqueous ethanol solution. Since it has been reported that Nalmefene, clinically confirmed to suppress impulsive alcohol drinking behavior of alcohol dependent patients and enable control of alcohol intake, shows effect in this evaluation system (Alcohol & Alcoholism 2001;36:2-10), brexpiprazole also suppresses impulsive alcohol drinking behavior of alcohol dependent patients.

### Example 7

1) Measurement of nerve activation pattern of c-fos-GFP (Cellar oncogene FBJ osteosarcoma green fluorescent protein) mouse Measurement method: c-fos is an indirect marker for neuronal activity, which is expressed on activation of nerve cell. Using a transgenic mouse introduced with a green fluorescent protein (GFP) gene at the downstream of the promoter of c-fos gene (c-fos-GFP mouse), the nerve activation pattern in the brain was measured. Brexpiprazole or vehicle was administered, and the brain was isolated 3 hr later. GFP signals from serial sections of the whole brain were stored in a computer using a two-photon microscope and, after three-dimensional reconstruction, the nerve activation patterns of brexpiprazole (OPC-34712) and vehicle groups were quantitatively analyzed using the brain map information.

### 2) Preparation of drug and administration method

Brexpiprazole was suspended in distilled water containing 5% gum arabic, and orally administered to c-fos-GFP mouse.

### 3) Number of mice and dose setting

Five to seven mice were used. The dose of brexpiprazole was 0.3 and 1 mg/kg.

### 4) Statistical analysis

The significance level of the test was set to 5%. In the comparison between groups in each brain region, Tukey's multiple comparison test was conducted.

### 5) Results

The test results are shown in Fig. 4. The area with a significant increase in the GFP signal relative to the vehicle group is shown white.

Brexpiprazole significantly increased GFP signal in the medial prefrontal cortex (ACA: Anterior cingulate area, PL: Prelimbic area, IL: Infralimbic area) at 0.3 and 1 mg/kg.

The above results have confirmed that brexpiprazole activates the nerve cell in the medial prefrontal cortex.

### Industrial Applicability

Brexpiprazole or a salt thereof is useful as a prophylactic and/or therapeutic agent for behavioral and psychological symptoms associated with neurodegenerative disease or impulsive symptoms associated with mental disease.

This application is based on US provisional patent application Nos. 61/718,305 and 61/782,467, the contents of which are incorporated by reference in full herein.

## Claims

1. A prophylactic and/or therapeutic agent comprising 7-[4-(4-benzo[b]thiophen-4-yl-piperazin-1-yl)butoxy]-1H-quinolin-2-one or a salt thereof as an active ingredient, for use in the prophylaxis or treatment of behavioral and psychological symptoms associated with neurodegenerative disease or impulsive symptoms associated with mental disease.

2. The prophylactic and/or therapeutic agent for use according to claim 1, which is a prophylactic and/or therapeutic agent for behavioral and psychological symptoms associated with neurodegenerative disease.

3. The prophylactic and/or therapeutic agent for use according to claim 1, which is a prophylactic and/or therapeutic agent for impulsive symptoms associated with mental disease.

4. The prophylactic and/or therapeutic agent for use according to claim 2, wherein the neurodegenerative disease is selected from the group consisting of dementia, multiple sclerosis, Parkinson syndrome, juvenile parkinsonism, striatonigral degeneration, progressive supranuclear palsy, pure akinesia, prion disease, corticobasal degeneration, chorea-acanthocytosis, benign hereditary chorea, paroxysmal choreoathetosis, essential tremor, essential myoclonus, Gilles de la Tourette syndrome, Rett syndrome, degenerative ballism, dystonia musculorum deformans, athetosis, spasmodic torticollis, Meige syndrome, cerebral palsy, Wilson's disease, Segawa's disease, Hallervorden-Spatz syndrome, neuroaxonal dystrophy, pallidal atrophy, spinocerebellar degeneration, cerebral cortical atrophy, Holmes-type cerebellar atrophy, olivopontocerebellar atrophy, hereditary olivopontocerebellar atrophy, Joseph disease, dentatorubropallidoluysian atrophy, Gerstmann-Straussler-Scheinker syndrome, Friedreich ataxia, Roussy-Levy syndrome, May-White syndrome, congenital cerebellar ataxia, periodic hereditary ataxia, ataxia telangiectasia, amyotrophic lateral sclerosis, progressive bulbar palsy, spinal progressive muscular atrophy, spinobulbar muscular atrophy, Werdnig-Hoffmann disease, Kugelberg-Welander disease, hereditary spastic paraplegia, syringomyelia, syringobulbia, Arnold-Chiari malformation, stiff man syndrome, Klippel-Feil syndrome, Fazio-Londe disease, low myelopathy, Dandy-Walker syndrome, spina bifida, Sjogren-Larsson syndrome, radiation myelopathy, age-related macular degeneration, and cerebral apoplexy due to cerebral hemorrhage and/or dysfunction or neurologic deficits associated therewith.

5. The prophylactic and/or therapeutic agent for use according to claim 4, wherein the neurodegenerative disease is dementia.

6. The prophylactic and/or therapeutic agent for use according to claim 5, wherein the dementia is Alzheimer-type dementia.

7. The prophylactic and/or therapeutic agent for use according to claim 5, wherein the dementia is dementia with Lewy bodies.

8. The prophylactic and/or therapeutic agent for use according to claim 5, wherein the dementia is frontotemporal dementia.

9. The prophylactic and/or therapeutic agent for use according to claim 5, wherein the dementia is cerebrovascular dementia.

10. The prophylactic and/or therapeutic agent for use according to claim 5, wherein the dementia is Parkinson's type dementia.

11. The prophylactic and/or therapeutic agent for use according to claim 5, wherein the dementia is Huntington's disease.

12. The prophylactic and/or therapeutic agent for use according to claim 4, wherein the neurodegenerative disease is multiple sclerosis.

13. The prophylactic and/or therapeutic agent for use according to claim 3, wherein the mental disease is selected from the group consisting of schizophrenia, treatment-resistant schizophrenia, refractory schizophrenia, chronic schizophrenia, emotional disturbance, psychotic disorder, mood disorder, bipolar disorder, mania, depression, endogenous depression, major depression, melancholic and treatment-resistant depression, dysthymic disorder, cyclothymic disorder, anxiety disorder, somatoform disorder, factitious disorder, dissociative disorder, sexual disorder, eating disorder, sleep disorder, adjustment disorder, substance-related disorder, anhedonia, delirium, vomiting, motion sickness, obesity, migraine, pain, mental retardation, autism, Tourette's disorder, tic disorder, attention deficit hyperactivity disorder, conduct disorder, intermittent explosive disorder, kleptomania, pyromania, pathological gambling, trichotillomania, Down's syndrome and personality disorder.

14. The prophylactic and/or therapeutic agent for use according to claim 13, wherein the mental disease is selected from the group consisting of schizophrenia, treatment-resistant schizophrenia, refractory schizophrenia and chronic schizophrenia.

15. The prophylactic and/or therapeutic agent for use according to claim 13, wherein the mental disease is selected from the group consisting of depression, endogenous depression, major depression, melancholic and treatment-resistant depression.

16. The prophylactic and/or therapeutic agent for use according to claim 13, wherein the mental disease is bipolar disorder.

17. The prophylactic and/or therapeutic agent for use according to claim 13, wherein the mental disease is eating disorder.

18. The prophylactic and/or therapeutic agent for use according to claim 13, wherein the mental disease is attention deficit hyperactivity disorder.

19. The prophylactic and/or therapeutic agent for use according to claim 13, wherein the mental disease is anxiety disorder.

20. The prophylactic and/or therapeutic agent for use according to claim 19, wherein the anxiety disorder is obsessive-compulsive disorder.

21. The prophylactic and/or therapeutic agent for use according to claim 19, wherein the anxiety disorder is post-traumatic stress disorder.

22. The prophylactic and/or therapeutic agent for use according to any one of claims 1 to 21, which is for use in the treatment of a patient who cannot receive a sufficient effect for behavioral and psychological symptoms associated with neurodegenerative disease or impulsive symptoms associated with mental disease from a generally available antipsychotic agent or therapeutic drug for neurodegenerative disease.

23. The prophylactic and/or therapeutic agent for use according to claim 22, wherein the generally available antipsychotic agent is chlorpromazine, fluphenazine, levomepromazine, perphenazine, propericiazine, bromperidol, haloperidol, pipamperone, timiperone, nemonapride, sulpiride, sultopride, carpipramine, clocapramine, mosapramine, pimozide, oxypertine, zotepine, amisulpride, risperidone, iloperidone, perospirone, paliperidone, lurasidone, ziprasidone, asenapine, clozapine, olanzapine, quetiapine, blonanserin, aripiprazole, cariprazine or sertindole, or a salt thereof.

24. The prophylactic and/or therapeutic agent for use according to claim 23, wherein the generally available therapeutic drug for neurodegenerative disease is donepezil, galantamine, rivastigmine, memantine, fingolimod, methylprednisolone, azathioprine, mitoxantrone, cyclophosphamide, interferon β preparation, glatiramer, teriflunomide or natalizumab, or a salt thereof.

25. 7-14-(4-Benzo[b]thiophen-4-yl-piperazin-1-yl)butoxy]-1H-quinolin-2-one or a salt thereof for use in the prophylaxis and/or treatment of behavioral and psychological symptoms associated with neurodegenerative disease or impulsive symptoms associated with mental disease.
